# EUROPÄISCHE PATENTSCHRIFT

(11) **EP 1 676 829 B1**
(45) Veröffentlichungstag und Bekanntmachung des Hinweises auf die Patenterteilung: **05.01.2011**
(21) Anmeldenummer: 05110310.9
(22) Anmeldetag: 03.11.2005
(51) Int. Cl.: C07C 67/303, C07C 69/14, C08K 5/12

(54) **Verfahren zur kontinuierlichen katalytischen Hydrierung**
Process for continuous catalytic hydrogenation
Procédé continu d'hydrogénation catalytique

(30) Priorität: 31.12.2004 DE 102004063673
(43) Veröffentlichungstag der Anmeldung: 05.07.2006
(73) Patentinhaber: Evonik Oxeno GmbH, 45772 Marl (DE)
(72) Erfinder: Graß, Michael Dr., 45721 Haltern am See (DE); Reeken, Bernhard, 46282 Dorsten (DE); Tuchlenski, Axel Dr., 69469 Weinheim (DE); Kaizik, Alfred Dr., 45772, Marl (DE)
(74) Vertreter: Hirsch, Hans-Ludwig

(56) Entgegenhaltungen:
- DE-A1- 10 203 386
- DE-A1- 10 225 565

## Beschreibung

Die Erfindung betrifft ein Verfahren zur kontinuierlichen katalytischen Hydrierung und insbesondere ein Verfahren zur Herstellung von alicyclischen Carbonsäuren oder deren Derivaten, insbesondere Carbonsäureestern durch Selektivhydrierung der entsprechenden aromatischen Carbonsäure(-derivate)n in mindestens drei hintereinandergeschalteten Reaktoren, wobei mindestens die beiden ersten in Schlaufenfahrweise betrieben werden.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine und für PVC Verwendung.

Für die Weichmachung von PVC werden überwiegend Ester der Phthalsäure, wie beispielweise Dibutyl-, Dioctyl-, Dinonyl- oder Didecylester der Phthalsäure, verwendet. Da über die Verwendung dieser Phthalate in letzter Zeit zunehmend kontrovers diskutiert wird, könnte deren Einsatz in Kunststoffen eingeschränkt werden. Alicyclische Polycarbonsäureester, von denen einige in der Literatur bereits als Weichmacher für Kunststoffe beschrieben sind, könnten dann als geeignete Ersatzstoffe Verwendung finden.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:

In US 5,286,898 und US 5,319,129 werden Verfahren beschrieben, mit denen Dimethylterephthalat an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

US 3,027,398 offenbart die Hydrierung von Dimethylterephthalat an geträgerten Ru-Katalysatoren bei 110 bis 140 °C und 35 bis 105 bar.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250 °C und 30 bis 200 bar hydriert. Dabei wird ein makroporöser Träger mit einer mittleren Porengröße von 70 nm und einer BET-Oberfläche von ca. 30 m²/g eingesetzt.

Weitere Ruthenium-Trägerkatalysatoren für die Herstellung von alicyclischen Polycarbonsäureestern durch Hydrierung von aromatischen Polycarbonsäureestern werden in den Schutzrechtsdokumenten WO 99/32427, WO 00/78704, DE 102 25 565.2 und DE 102 32 868.4 beansprucht.

In WO 2004/046078 wird die Hydrierung von Benzolpolycarbonsäuren oder deren Derivaten an einem Katalysator beschrieben, der das aktive Katalysatormetall aufgebracht auf einem Träger aufweist, wobei der ein oder mehrere Materialien mit geordneten Mesoporen aufweist.

Die Hydrierung der aromatischen Polycarbonsäureester erfolgt in US 3,027,398 ansatzweise, in US 5,286,898, US 5,319,129, DE 28 23 165, WO 99/32427 und WO 00/78704 kontinuierlich in einem Rohrreaktor ohne oder mit Rückführung (Schlaufenfahrweise) des Hydrieraustrags.

In DE 102 32 868.4 und DE 102 25 565.2 wird die Hydrierung der aromatischen Polycarbonsäureester zu den entsprechenden alicyclischen Polycarbonsäureestern in zwei hintereinandergeschalteten Reaktoren durchgeführt, wobei der erste in Schlaufenfahrweise (teilweise Rückführung des Reaktoraustrages) und der zweite im geraden Durchgang betrieben wird. Der erste Schlaufenreaktor kann auch durch mehrere kleine in Reihe oder parallel verschaltete Schlaufenreaktoren ersetzt werden, wobei diese Reaktoren einen gemeinsamen Kreislauf aufweisen.

Die technisch bekannten Verfahren können hinsichtlich der Raum-Zeit-Ausbeute und/oder der Selektivität nicht voll befriedigen. Zudem werden relativ große Mengen an Katalysator benötigt. Aufgabe der vorliegenden Erfindung bestand deshalb in der Bereitstellung eines Hydrierverfahrens, welches mit möglichst wenig Katalysator bezogen auf den zu erzielenden Umsatz durchgeführt werden kann.

Es wurde nun gefunden, dass das benötigte Katalysatorvolumen bei der Hydrierung, insbesondere bei der Kernhydrierung von aromatischen Carbonsäureestern zu den entsprechenden alicyclischen Carbonsäureestern minimiert werden kann, wenn die Hydrierung in mindestens drei hintereinander geschalteten Hydriereinheiten durchgeführt wird, wobei zumindest die beiden ersten Hydriereinheiten in Schlaufenfahrweise, d. h. unter Rückführung eines Teils des jeweiligen Hydrieraustrags und unter Verwendung von bestimmten Katalysatorvolumina in den einzelnen Hydriereinheiten betrieben werden.

Gegenstand der vorliegenden Erfindung ist deshalb ein Verfahren zur kontinuierlichen katalytischen Hydrierung von hydrierbaren Verbindungen an festen im Festbett angeordneten Katalysatoren mit einem wasserstoffhaltigen Gas, gemäß Anspruch 1,
welches dadurch gekennzeichnet ist, dass die Hydrierung in mindestens drei hintereinandergeschalteten Hydriereinheiten durchgeführt werden und dass mindestens die beiden ersten Hydriereinheiten in Schlaufenfahrweise betrieben wird, wobei in den Hydriereinheiten Katalysatorvolumina verwendet werden, die um maximal 20 % von den Katalysatorvolumina abweichen, die erhältlich sind durch
a) Bestimmung der Kinetik der durchzuführenden Hydrierung,
b) Berechnen des erforderlichen Katalysatorvolumens für die eingesetzten Reaktortypen bei vorgegebenen Reaktorein- und -ausgangskonzentrationen,
c) Ermitteln des erforderlichen Gesamtkatalysatorvolumens durch Kombination der berechneten Katalysatorvolumina, wobei jeweils nur solche Kombinationen vorgenommen werden, die zur gewünschten Endkonzentration des eingesetzten zu hydrierenden Eduktes führen,
d) Erstellen einer Kurve aus den in c) ermittelten Gesamtkatalysatorvolumina aufgetragen gegen den Umsatz,
e) Bestimmen des Minimums der in d) erstellten Kurve und
f) Ermitteln der dem Minimum zuzuordnenden Katalysatorvolumina der einzelnen Hydriereinheiten.

Unter einer Hydriereinheit wird hier und im folgenden Text ein Hydrierreaktor oder mehrere hintereinandergeschalteter Reaktoren oder mehrere parallel zueinandergeschalteten Reaktoren oder eine Reaktorgruppe, die aus parallel und hintereinander geschalteten Reaktoren besteht, verstanden, also ein Reaktor oder eine Reaktoranordnung, die im erfindungsgemäßen Verfahren die Funktion eines Reaktors ausüben kann.

Das erfindungsgemäße Verfahren hat den Vorteil, dass das bei Hydrierungsreaktionen einzusetzende Katalysatorvolumen in Bezug auf den Umsatz minimiert werden kann. Auf diese Weise können die Beschaffungskosten für Ersatzbefüllungen an Katalysator sowie die Reaktorgröße auf das notwendige begrenzt werden.

Durch die einfache Hintereinanderschaltung von zumindest zwei Schlaufenreaktoren kann die Raum-Zeit-Ausbeute im Vergleich mit den im Stand der Technik beschriebenen Verfahren außerdem erhöht werden. Durch das Vorhandensein von mehreren Schlaufenreaktoren können außerdem eine höhere Ausfallsicherheit, eine einfachere Wartung und höhere Standzeiten erreicht werden.

Das erfindungsgemäße Verfahren wird nachfolgend beispielhaft beschrieben, ohne dass die Erfindung auf die beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können.

Das erfindungsgemäße Verfahren zur kontinuierlichen katalytischen Hydrierung von hydrierbaren Verbindungen an festen im Festbett angeordneten Katalysatoren mit einem Wasserstoff-haltigen Gas, zeichnet sich dadurch aus, dass die Hydrierung in mindestens drei hintereinandergeschalteten Hydriereinheiten durchgeführt wird und dass mindestens die beiden ersten Hydriereinheiten in Schlaufenfahrweise betrieben werden, wobei in den Hydriereinheiten Katalysatorvolumina verwendet werden, die um maximal 20 %, vorzugsweise um maximal 10 %, bevorzugt um maximal 5 % und ganz besonders bevorzugt um maximal 2 % von den Katalysatorvolumina abweichen, die erhältlich sind durch
a) Bestimmung der Kinetik der durchzuführenden Hydrierung,
b) Berechnen des erforderlichen Katalysatorvolumens für die eingesetzten Reaktortypen bei vorgegebenen Reaktorein- und -ausgangskonzentrationen,
c) Ermitteln des erforderlichen Gesamtkatalysatorvolumens durch Kombination der berechneten Katalysatorvolumina, wobei jeweils nur solche Kombinationen vorgenommen werden, die zur gewünschten Endkonzentration des eingesetzten zu hydrierenden Eduktes führen,
d) Erstellen einer Kurve aus den in c) ermittelten Gesamtkatalysatorvolumina aufgetragen gegen den Umsatz,
e) Bestimmen des Minimums der in d) erstellten Kurve und
f) Ermitteln der dem Minimum zuzuordnenden Katalysatorvolumina der einzelnen Hydriereinheiten.
Durch die Durchführung des Verfahrens mit den so berechneten Katalysatorvolumina kann das benötigte Katalysatorvolumen bezogen auf den Umsatz möglichst gering gehalten werden.

Die Bestimmung der Kinetik der durchzuführenden Hydrierung kann durch alle erdenklichen, dem Fachmann bekannten Methoden erfolgen. Bevorzugt wird zunächst der für die zu betrachtende Hydrierung einzusetzende Katalysator festgelegt. Geeignete Katalysatoren können im Stand der Technik gefunden werden oder aber durch eine Katalysatorauswahl (Katalysatorscreening), z. B. mittels Hochdurchsatz-Methoden bestimmt werden.

Nach der Auswahl der Katalysatoren werden Versuche zur Bestimmung der Reaktionsgeschwindigkeit durchgeführt, wobei die die Reaktionsgeschwindigkeit beeinflussenden physikalischen Größen wie z. B. Temperatur, Druck, Konzentration (zu Beginn der Reaktion) und LHSV (Volumenstrom Edukt bezogen auf Katalysatorvolumen) variiert werden. Die Anzahl der durchzuführenden Versuche ergibt sich aus der statistischen Versuchsplanung (Design of Experiments, DoE).

An Hand der durch die Versuche ermittelten Daten erfolgt mittels einer Auswertesoftware eine Anpassung an ein kinetisches Modell, welches alle relevanten Reaktionen und Nebenreaktionen im Reaktionsraum beschreibt und die realen Temperatur- und Druckabhängigkeiten berücksichtigt. Als Auswertesoftware kann z. B. das Software-Paket Presto-Kinetics der Dr. Michael Wulkow Computing in Technology GmbH (CiT), Oldenburger Str. 200, D-26180 Rastede Germany verwendet werden. Für das jeweilige kinetische Modell werden die relevanten Parameter und Konstanten, wie z. B. Reaktionsordnung, Geschwindigkeitskonstante und Aktivierungsenergie angegeben und durch die Auswertesoftware optimiert. Die Anpassung kann als erfolgt angesehen werden, wenn die Abweichung (Residuum) zwischen Modell und den tatsächlich ermittelten Daten kleiner 20 %, vorzugsweise kleiner 10 %, bevorzugt kleiner 7,5 % und besonders bevorzugt kleiner 5 % beträgt. Werden während der Optimierung/Anpassung nur größere Abweichungen erreicht, muss das Modell entsprechend angepasst werden. Gegebenenfalls müssen weitere für das Modell notwendige Daten durch weitere Versuche generiert werden. So können z. B. je nach betrachtetem Reaktionssystem Adsorptionskonstanten und/oder weitere Parameter (druckabhängige Gaslöslichkeiten o. ä.) erhalten werden bzw. erforderlich sein.

Mit der so erhaltenen Kinetik kann im Schritt b) das erforderliche Katalysatorvolumen für die zum Einsatz vorgesehenen Reaktortypen bei vorgegebenen Reaktorein- und - ausgangskonzentrationen für die verwendeten Reaktoren (und damit definiertem bzw. vorgegebenem Umsatz), bei ansonsten konstanten Parametern (Temperatur, Druck etc.) berechnet werden. Die Berechnung kann wiederum mit dem Software-Paket Presto-Kinetics erfolgen. Als Reaktortypen kommen alle gängigen Reaktortypen in Frage, wie z. B. Rohrreaktoren, Kreislaufreaktoren und Rührkessel sowie Kaskadierung der Reaktortypen.

An Hand der gemäß b) berechneten Katalysatorvolumina kann durch sinnvolle Kombination von Einzelvolumina, wobei jeweils nur solche Kombinationen vorgenommen werden, die zur gewünschten Endkonzentration des eingesetzten zu hydrierenden Eduktes führen, ein benötigtes Gesamtkatalysatorvolumen bestimmt werden (Schritt c)). Das Gesamtkatalysatorvolumen ergibt sich aus der Addition der Einzelvolumina.

In dem nächsten Schritt d) wird aus den in c) ermittelten Gesamtkatalysatorvolumina aufgetragen gegen den Umsatz, eine Kurve ermittelt. Durch Bestimmung des Minimums der Kurve in Schritt e), die graphisch oder programmgestützt erfolgen kann, kann das optimale Gesamtkatalysatorvolumen bestimmt werden.

Aus dem Gesamtkatalysatorvolumen für den Punkt im Minimum kann in Schritt f) die entsprechende Aufteilung des Katalysatorvolumens auf die beteiligten Reaktoren entnommen werden. Über die jeweiligen bestimmten Katalysatorvolumina kann die optimale zugehörige Verweilzeit bestimmt werden. Unter Verweilzeiten werden im Rahmen der vorliegenden Erfindung mittlere Verweilzeiten (reziproke LHSV) verstanden. Diese sind definiert als der Quotient des gegebenen Reaktionsvolumens bzw. des Schüttvolumens des Katalysators (ist bei heterogenen Reaktionssystemen gleich zu setzen) und dem Gesamtvolumenstrom (ohne Recycelstrom) des Einsatzstoffes (des zu hydrierendem Edukts).

Nachfolgend soll die Durchführung des Schrittes b) an Hand eines 3-stufigen Verfahren (zwei Schlaufenreaktoren und ein Rohrreaktor im geraden Durchgang) zur Kernhydrierung von Diisononylphthalaten (DINP) beispielhaft beschrieben werden, ohne dass das erfindungsgemäße Verfahren auf diese Ausführungsform beschränkt sein soll. Für die erste Reaktionsstufe (Schlaufenreaktor) wurde bei fester Eintrittskonzentration (100 Massen-% DINP) die Endkonzentration variiert und die abhängigen Katalysatorvolumina berechnet. Für die zweite Reaktionsstufe (zweiter Schlaufenreaktor) wurde mit einer festen Endkonzentration (5 Massen-% DINP) das abhängige Katalysatorvolumen zu den variierenden Eintrittskonzentrationen berechnet. Das Katalysatorvolumen für die dritte Reaktionsstufe (Rohrreaktor im geraden Durchgang zur Finishinghydrierung) wurde durch Nebenbedingungen wie Exothermie auf maximalen Umsatz von 5 % limitiert um eine adiabate Fahrweise realisieren zu können. Bei einem Kreislaufreaktor hängt das aus der Kinetik resultierende Katalysatorvolumen von der Eintrittskonzentration und dem Umsatz ab. Die Ergebnisse der Durchführung der Anpassung des Modells können Beispiel 1 entnommen werden.

Das Verhältnis der Verweilzeiten in den in Reihe hintereinandergeschalteten Schlaufenreaktoren beträgt im erfindungsgemäßen Verfahren vorzugsweise von 0,01 bis kleiner 1, bevorzugt von 0,1 bis 0,9 und besonders bevorzugt von 0,2 bis 0,5. Die Verweilzeiten werden vorzugsweise so eingestellt, dass in dem ersten der hintereinandergeschalteten Schlaufenreaktoren ein Umsatz von 40 bis 90 %, bevorzugt von 60 bis 90 % und im zweiten Schlaufenreaktor ein Umsatz von 2 bis 60 %, bevorzugt von 5 bis 40 % bezogen auf die Ausgangskonzentration der zu hydrierenden Verbindung am Eingang des jeweiligen Reaktors erzielt wird.

Es kann vorteilhaft sein, wenn alle Hydriereinheiten im erfindungsgemäßen Verfahren in Schlaufenfahrweise betrieben werden. Ebenso vorteilhaft kann es sein, wenn die letzte Hydriereinheit im geraden Durchgang betrieben wird. Das erfindungsgemäße Verfahren wird in einer Vorrichtung durchgeführt, die mindestens drei Hydriereinheiten aufweist.

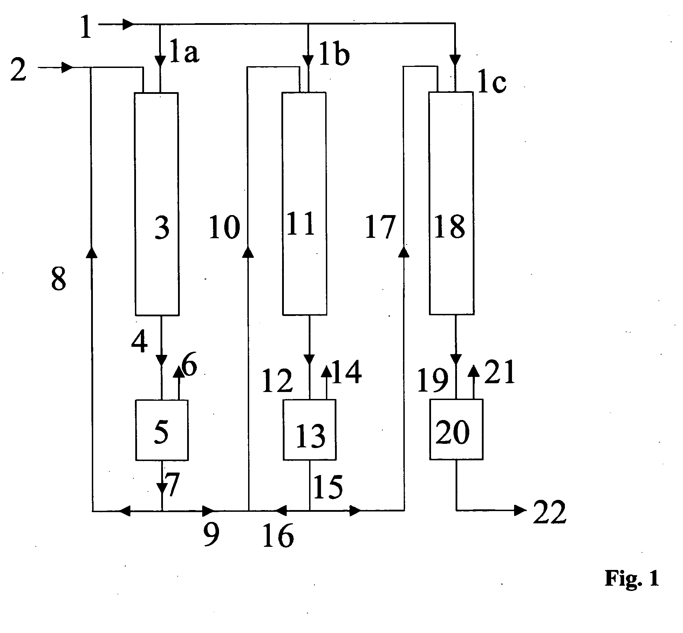

Eine Variante des erfindungsgemäßen Verfahrens mit drei Hydriereinheiten ist als Blockschema in Fig. 1 dargestellt. Es sei betont, dass die hier dargestellte Variante sinngemäß auch für Verfahren mit mehr als drei Hydriereinheiten gilt. In der in Fig. 1 dargestellten Ausführungsform der Erfindung werden die ersten beiden Hydriereinheiten in Schlaufenfahrweise und die dritte Hydriereinheit im geraden Durchgang betrieben. Es sind weitere Ausführungsformen möglich, bei denen alle drei Hydriereinheiten in Schlaufenfahrweise betrieben werden oder bei denen mehr als drei Hydriereinheiten vorhanden sind. Wird die Hydrierung in einer Hydrieranlage mit mehr als drei Hydriereinheiten durchgeführt, gilt gemäß der Erfindung, dass die ersten beiden Hydriereinheiten in Schlaufenfahrweise betrieben werden und die folgenden Hydriereinheiten wahlweise in Schlaufenfahrweise oder im geraden Durchgang betrieben werden können.

In der in Fig. 1 dargestellten Variante des erfindungsgemäßen Verfahrens wird jeder einzelne Reaktor mit Hydriergas beschickt. Um den Wasserstoffverbrauch und die durch die Abgasströme bedingten Austragsverluste zu minimieren, kann es zweckmäßig sein, das Abgas einer Hydriereinheit als Hydriergas für eine andere Hydriereinheit zu verwenden. Beispielweise kann in einem Verfahren wie in Fig. 1 dargestellt das Abgas (6) aus der ersten Hydriereinheit (3) anstelle des Hydriergases (1b) in die zweite Hydriereinheit (11) und das Abgas (14) der zweiten Hydriereinheit (11) anstelle des Hydriergases (1c) in die dritte Hydriereinheit (18) eingespeist werden. In diesem Falle strömen flüssige Edukt/Produkt-Phase und Hydriergas in gleicher Reihenfolge durch die Reaktoren. Ebenso kann es zweckmäßig sein, Hydriergas und Edukt/Produkt-Phase in entgegengesetzter Richtung durch die Reaktoren strömen zu lassen. In diesem Falle wird frisches Hydriergas in den letzten Reaktor eingeleitet und Abgas aus dem ersten Reaktor abgeleitet. Weiterhin können zwei oder mehrere Reaktoren ein gemeinsames Hydriergassystem besitzen und andere Reaktoren davon getrennt mit Hydriergas beschickt werden. Bei der Verwendung des Abgases eines Reaktors als Hydriergas eines anderen Reaktors kann, falls gewünscht, der Druckverlust durch Zwischenkompression ausgeglichen werden.

Vorzugsweise werden die Abgasmengen bzw. Gasströme so eingestellt, dass in allen Reaktoren eine gute Fluiddynamik vorliegt. Eine gute Fluiddynamik zeichnet sich dadurch aus, dass eine geringe Wandströmung, eine hohe Grenzfläche zum Stoffaustausch und/oder Pulse-Flow (pulsierender Fluss) vorliegt.

Als Hydriergase können beliebige wasserstoffhaltige Gasgemische, die keine schädlichen Mengen an Katalysatorgiften, wie beispielsweise Kohlenmonoxid oder Schwefelwasserstoff enthalten, eingesetzt werden. Die Verwendung von Inertgasen ist optional, bevorzugt wird Wasserstoff in einer Reinheit größer 95 %, insbesondere größer 98 % eingesetzt. Inertgasanteile können beispielsweise Stickstoff oder Methan sein. Vorzugsweise ist in den Hydriereinheiten soviel Wasserstoff vorhanden, dass dieser im Überschuss, insbesondere in einem Überschuss von 200 %, bevorzugt in einem Überschuss von 5 bis 100 % und besonders bevorzugt in einem Überschuss von 10 bis 50 % bezogen auf die stöchiometrische Menge, die zur Erzielung des in der Hydriereinheit möglichen bzw. gewünschten Umsatzes benötigt wird, vorliegt. Ohne das Einstellen eines ausreichenden Überschusses an Wasserstoff gelingt die Hydrierung der aromatischen Bindungen nur unvollkommen, was zu Ausbeuteverlusten führt.

Als hydrierbare Verbindungen können in dem erfindungsgemäßen Verfahren aromatische Carbonsäuren oder deren Derivate eingesetzt werden, die kernhydriert werden.

Im erfindungsgemäßen Verfahren kann insbesondere eine aromatische Carbonsäure oder ein Derivat davon oder ein Gemisch aromatischer Carbonsäuren oder deren Derivate in der Flüssigphase oder Flüssig/Gas-Mischphase kontinuierlich an einem im Festbett angeordneten Katalysator in mindestens drei hintereinandergeschalteten Hydriereinheiten mit Wasserstoff zu dem/den entsprechenden alicyclischen Carbonsäuren bzw. deren Derivaten hydriert werden.

Mit dem erfindungsgemäßen Verfahren können aromatische Carbonsäuren oder deren Derivate, wie aromatische Mono-, Di- oder Polycarbonsäuren oder deren Derivate, wie z. B. deren Ester oder Anhydride, insbesondere deren Alkylester, zu den entsprechenden alicyclischen Carbonsäureverbindungen umgesetzt werden. Dabei können als aromatische Di- oder Polycarbonsäurederivate sowohl Vollester als auch Partialester mit dem erfindungsgemäßen Verfahren hydriert werden. Unter Vollester wird eine Verbindung verstanden, bei der alle Säuregruppen verestert sind. Partialester sind Verbindungen mit mindestens einer freien Säuregruppe (oder gegebenenfalls einer Anhydridgruppe) und mindestens einer Estergruppe. Werden im erfindungsgemäßen Verfahren Polycarbonsäureester eingesetzt, so enthalten diese bevorzugt 2, 3 oder 4 Esterfunktionen.

Im erfindungsgemäßen Verfahren können als aromatische Di- oder Polycarbonsäuren oder deren Derivate bevorzugt Benzol-, Diphenyl-, Naphthalin-, Diphenyloxid- oder Anthracenpolycarbonsäuren, deren Anhydride und/oder die entsprechenden Ester eingesetzt werden. Die durch das erfindungsgemäße Verfahren erhaltenen alicyclischen Di- oder Polycarbonsäuren bzw. deren Derivate bestehen aus einem oder mehreren, gegebenenfalls durch eine C-C-Bindung verknüpfte oder ankondensierte C₆-Ringen.

In einer bevorzugten Ausführungsform betritt die vorliegende Erfindung ein Verfahren zur Hydrierung der 1,2-, 1,3- oder 1,4-Benzoldicarbonsäure oder deren Derivate, insbesondere deren Ester, und/oder der 1,2,3-, 1,2,4- oder 1,3,5-Benzoltricarbonsäure oder deren Derivate, insbesondere deren Ester, d. h. es werden die Isomere der 1,2-, 1,3- oder 1,4-Cyclohexandicarbonsäure oder deren Derivate, insbesondere deren Ester, oder der 1,2,3-, 1,3,5- oder 1,2,4-Cyclohexantricarbonsäure oder deren Derivate, insbesondere deren Ester erhalten.

Im erfindungsgemäßen Verfahren können beispielsweise folgende aromatische Carbonsäuren oder deren Derivate, insbesondere Ester eingesetzt werden: 1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure. Weiterhin können Säuren oder deren Derivate, insbesondere Ester eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.

Als aromatische Monocarbonsäuren oder deren Derivate können in dem erfindungsgemäßen Verfahren z. B. Benzoesäure, 1-Naphthoesäure oder 2-Naphthoesäure oder deren Derivate, insbesondere deren Ester eingesetzt werden. Weiterhin können Monocarbonsäuren oder deren Derivate, insbesondere Ester eingesetzt werden, die aus den genannten Monocarbonsäuren durch Substitution eines oder mehrerer am aromatischen Kern gebundenen Wasserstoffatome durch Alkyl-, Cycloalkyl- oder Alkoxyalkylgruppen entstehen.

Besonders bevorzugt werden in dem erfindungsgemäßen Verfahren die aromatischen Carbonsäureester der vorgenannten aromatischen Carbonsäuren eingesetzt. Die Alkoholkomponente der bevorzugt eingesetzten aromatischen Carbonsäureester besteht bevorzugt aus verzweigten oder linearen (unverzweigten) Alkyl-, Cycloalkyl- oder Alkoxyalkylgruppen mit 1 bis 25 Kohlenstoffatomen, vorzugsweise 3 bis 15, besonders bevorzugt 8 bis 13 C-Atomen und ganz besonders bevorzugt 9 oder 10 C-Atomen. Die Alkoholkomponente kann eine oder mehrere Hydroxy-Gruppen aufweisen. Sind in einem Molekül mehr als eine Carboxygruppe vorhanden, so können die Alkoholkomponenten in einem Molekül eines eingesetzten aromatischen Polycarbonsäureesters gleich oder unterschiedlich sein, d. h. sie können gleiche oder verschiedene Isomeren oder Kettenlängen besitzen. Selbstverständlich können auch Isomere bezüglich des Substitutionsmusters des aromatischen Systems in Form eines Gemisches eingesetzt werden, z. B. ein Gemisch aus Phthalsäureester und Terephthalsäureester.

Im erfindungsgemäßen Verfahren können als Ester einer aromatischen Di- oder Polycarbonsäure beispielsweise folgende Verbindungen eingesetzt werden: Terephthalsäuremonomethylester, Terephthalsäuredimethylester, Terephthalsäurediethylester, Terephthalsäuredi-n-propylester, Terephthalsäuredibutylester, Terephthalsäurediisobutylester, Terephthalsäuredi-tert.-butylester, Terephthalsäuremonoglykolester, Terephthalsäurediglykolester, Terephthalsäure-diisoheptylester, Terephthalsäure-n-octylester, Terephthalsäurediisooctylester, Terephthalsäuredi-2-ethylhexylester, Terephthalsäuredi-n-nonylester, Terephthalsäurediisononylester, Terephthalsäuredi-n-decylester, Terephthalsäurediisodecylester, Terephthalsäuredipropylheptylester, Terephthalsäuredi-n-undecylester, Terephthalsäurediisododecylester, Terephthalsäure-ditridecylester, Terephthalsäuredi-n-octadecylester, Terephthalsäurediisooctadecylester, Terephthalsäuredi-n-eicosylester, Terephthalsäuremonocyclohexylester; Phthalsäuremonomethylester, Phthalsäure-dimethylester, Phthalsäuredi-n-propylester, Phthalsäuredi-n-butylester, Phthalsäure-diisobutylester, Phthalsäuredi-tert.-butylester, Phthalsäuremonoglykolester, Phthalsäure-diglykolester, Phthalsäurediisoheptylester, Phthalsäuredi-n-octylester, Phthalsäure-diisooctylester, Phthalsäurediethylhexylester, Phthalsäuredi-n-nonylester, Phthalsäure-diisononylester, Phthalsäuredi-n-decylester, Phthalsäuredi-2-propylheptylester, Phthalsäure-diisodecylester, Phthalsäuredi-n-undecylester, Phthalsäurediisoundecylester, Phthalsäure-ditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäuredimethylester, Isophthalsäurediethylester, Isophthalsäuredi-n-propylester, Isophthalsäuredi-n-butylester, Isophthalsäurediisobutylester, Isophthalsäuredi-tert.-butylester, Isophthalsäuremonoglykolester, Isophthalsäurediglykolester, Isophthalsäurediisoheptylester, Isophthalsäuredi-n-octylester, Isophthalsäurediisooctylester, Isophthalsäuredi-2-ethylhexylester, Isophthalsäuredi-n-nonylester, Isophthalsäurediisononylester, Isophthalsäuredi-n-decylester, Isophthalsäurediisodecylester, Isophthalsäuredipropylheptylester Isophthalsäuredi-n-undecylester, Isophthalsäurediisododecylester, Isophthalsäuredi-n-dodecylester, Isophthalsäure-ditridecylester, Isophthalsäuredi-n-octadecylester, Isophthalsäurediisooctadecylester, Isophthalsäuredi-n-eicosylester, Isophthalsäuremonocyclohexylester. Als aromatisches Dicarbonsäurederivat wird ganz besonders bevorzugt Diisononylphthalat oder Didecylphthalat eingesetzt.

Im erfindungsgemäßen Verfahren können als Ester der Monocarbonsäuren z. B. Benzoate von Diolen, wie beispielsweise Glycoldibenzoat, Diethylenglycoldibenzoat, Triethylenglycoldibenzoat oder Dipropylenglycoldibenzoat, aber auch Benzoesäurealkylester, wie z. B. Decyl- oder Isodecylbenzoat, Nonyl- oder Isononylbenzoat, Octyl- oder Isooctylbenzoat, 2-Ethylhexylbenzoat oder Tridecyl- oder Isotridecylbenzoat eingesetzt werden. Als aromatisches Monocarbonsäurederivat wird besonders bevorzugt Isononylbenzoat oder Decylbenzoat eingesetzt.

In dem erfindungsgemäßen Verfahren können auch Gemische aus zwei oder mehreren Carbonsäuren oder Carbonsäurederivaten, insbesondere Gemische von Carbonsäureestern eingesetzt werden. Solche Gemische können beispielsweise auf folgenden Wegen erhalten werden:
a) Eine Di- oder Polycarbonsäure wird mit einem Alkohol derart partiell verestert, dass Voll- und Partialester nebeneinander vorliegen.
b) Ein Gemisch von mindestens zwei Carbonsäuren wird mit einem Alkohol verestert, wobei ein Gemisch von mindestens zwei Vollestern entsteht.
c) Eine Di- oder Polycarbonsäure wird mit einem Alkoholgemisch versetzt, wobei ein entsprechendes Gemisch der Vollester entstehen kann.
d) Eine Di- oder Polycarbonsäure wird mit einem Alkoholgemisch partiell verestert.
e) Ein Gemisch von mindestens zwei Carbonsäuren wird mit einem Alkoholgemisch partiell verestert.
f) Ein Gemisch aus mindestens zwei Di- oder Polycarbonsäuren wird mit einem Alkoholgemisch partiell verestert.

Bei diesen Umsetzungen können an Stelle der Polycarbonsäuren auch die entsprechenden Anhydride eingesetzt werden.

Großtechnisch werden aromatische Ester, insbesondere die Vollester auf Weg c) häufig aus Alkoholgemischen hergestellt. Entsprechende Alkoholgemische sind beispielsweise: C₅-Alkoholgemische, hergestellt aus linearen Butenen durch Hydroformylierung und anschließender Hydrierung;
C₅-Alkoholgemische, hergestellt aus Isobuten oder Butengemischen, die lineare Butene und Isobuten enthalten, durch Hydroformylierung und anschließende Hydrierung; C₆-Alkoholgemische, hergestellt aus einem Penten oder aus einem Gemisch von zwei oder mehreren Pentenen, durch Hydroformylierung und anschließende Hydrierung; C₇-Alkoholgemische, hergestellt aus Triethylen oder Dipropen oder einem Hexenisomer oder einem sonstigen Gemisch von Hexenisomeren, durch Hydroformylierung und anschließende Hydrierung;
C₈-Alkoholgemische, wie 2-Ethylhexanol (2 Isomere), hergestellt durch Aldolkondensation von n-Butyraldehyd und anschließende Hydrierung;
C₉-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher ein Vielzahl von technischen C₉-Alkoholgemischen.
C₁₀-Alkoholgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung;
C₁₀-Alkoholgeinische, hergestellt aus einem Gemisch von mindestens zwei C₅-Aldehyden durch Aldolkondensation und anschließende Hydrierung;
C₁₃-Alkohollgemische, hergestellt aus Dihexen, Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen. Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Carbonsäuren und den oben genannten Alkoholgemischen, eingesetzt werden. Erfindungsgemäß werden bevorzugt Ester, hergestellt aus Phthalsäure, Phthalsäureanhydrid oder Benzoesäure und einem Gemisch isomerer Alkohole mit 6 bis 13 C-Atomen, eingesetzt.

Beispiele für technische Phthalate, die im erfindungsgemäßen Verfahren eingesetzt werden können, sind folgende Produkte mit den Handelsnamen:
Vestinol C (Di-n-butylphthalat) (CAS Nr.84-74-2); Vestinol IB (Di-i-butylphthalat) (CAS Nr. 84-69-5); Jayflex DINP (CAS Nr.68515-48-0); Jayflex DIDP (CAS Nr.68515-49-1); Palatinol 9P (68515-45-7), Vestinol 9 (CAS Nr. 28553-12-0); TOTM (CAS Nr. 3319-31-1); Linplast 68-TM , Palatinol N (CAS Nr. 28553-12-0); Jayflex DHP (CAS Nr. 68515-50-4); Jayflex DIOP (CAS Nr. 27554-26-3); Jayflex UDP (CAS Nr. 68515-47-9); Jayflex DIUP (CAS Nr. 85507-79-5); Jayflex DTDP (CAS Nr.68515-47-9); Jayflex L9P (CAS Nr. 68515-45-7); Jayflex L911P (CAS Nr. 68515-43-5); Jayflex L11P (CAS Nr. 3648-20-2); Witamol 110 (CAS Nr. 68515-51-5); Witamol 118 (Di-n-C8-C10-alkylphthalat) (CAS Nr.71662-46-9); Unimoll BB (CAS Nr. 85-68-7); Linplast 1012 BP (CAS Nr. 90193-92-3); Linplast 13XP (CAS Nr.27253-26-5); Linplast 610P (CAS Nr. 68515-51-5); Linplast 68 FP (CAS Nr. 68648-93-1); Linplast 812 HP (CAS Nr. 70693-30-0); Palatinol AH (CAS Nr. 117-81-7); Palatinol 711 (CAS Nr. 68515-42-4); Palatinol 911 (CAS Nr. 68515-43-5); Palatinol 11 (CAS Nr. 3648-20-2); Palatinol Z (CAS Nr.26761-40-0); Palatinol DIPP (CAS Nr. 84777-06-0); Jayflex 77 ( CAS Nr. 71888-89-6); Palatinol 10 P (CAS Nr. 533-54-0); Vestinol AH (CAS Nr. 117-81-7).

Es sei darauf hingewiesen, dass bei der Kernhydrierung aromatischer Di- oder Polycarbonsäuren bzw. ihrer Ester aus jedem eingesetzten Isomer mindestens zwei stereoisomere Hydrierprodukte entstehen können. Die Mengenverhältnisse der dabei entstandenen Stereoisomeren zueinander hängen vom verwendeten Katalysator und von den Hydrierbedingungen ab. Alle Hydrierprodukte mit beliebigen Verhältnis(sen) der Stereoisomeren zueinander können ohne Auftrennung oder aber nach einer Auftrennung verwendet werden. In der Regel werden die Hydrierprodukte ohne Auftrennung verwendet.

Im erfindungsgemäßen Verfahren werden feste Hydrierkatalysatoren eingesetzt, die vorzugsweise mindestens ein Metall der ersten, siebten und/oder achten Nebengruppe des Periodensystems der Elemente, bevorzugt mindestens ein Metall der achten Nebengruppe des Periodensystems der Elemente enthalten. Vorzugsweise werden, insbesondere bei der Hydrierung von aromatischen Carbonsäuren oder deren Derivaten, als Aktivmetalle der achten Nebengruppe des Periodensystems der Elemente Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird.

Neben den bereits genannten Metallen kann zusätzlich mindestens ein Metall der ersten und/oder siebten Nebengruppe des Periodensystems der Elemente in den Katalysatoren enthalten sein. Bevorzugt wird Rhenium und/oder Kupfer eingesetzt.

Der Gehalt der Aktivmetalle, d. h. der Metalle der ersten und/oder siebten und/oder achten Nebengruppe des Periodensystems der Elemente beträgt vorzugsweise 0,1 bis 30 Massen-%. Der Edelmetallgehalt, d. h. der Metalle der achten Nebengruppe des Periodensystems der Elemente und der fünften oder sechsten Periode, z. B. Palladium, Ruthenium, berechnet als Metall liegt vorzugsweise im Bereich von 0,1 bis 10 Massen-%, insbesondere im Bereich von 0,8 bis 5 Massen-%, ganz besonders zwischen 1 und 3 Massen-%. Die genannten Gehalte an Aktivmetallen sind insbesondere bei der Hydrierung von aromatischen Carbonsäuren oder deren Derivaten besonders bevorzugt.

Bevorzugt sind die eingesetzten Katalysatoren Trägerkatalysatoren. Als Träger können beispielsweise folgende Stoffe verwendet werden: Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische. Besonders bevorzugt wird ein Katalysator eingesetzt, der einen Titandioxid-Träger aufweist. Zusätzlich können diese Trägermaterialien Alkalimetalle, Erdalkalimetalle und/oder Schwefel enthalten.

Im erfindungsgemäßen Verfahren werden bevorzugt Ruthenium-Katalysatoren eingesetzt, die in den Patentschriften DE 102 25 565.2 und DE 102 32 868.4 beansprucht werden.

Im erfindungsgemäßen Verfahren bestehen die Hydriereinheiten vorzugsweise jeweils aus einem Hydrierreaktor. Dieser kann ein Rohrreaktor, Rohrbündelreaktor oder vorzugsweise ein Schachtofen sein.

Die einzelnen Reaktoren können adiabatisch, polytrop oder praktisch isotherm, d. h. mit einem Temperaturanstieg von typischerweise kleiner als 10 Kelvin betrieben werden. Dabei werden insbesondere die in Schlaufenfahrweise betriebenen Reaktoren bevorzugt quasi isotherm gefahren, vorzugsweise mit einem Temperaturanstieg kleiner 10 Kelvin besonders bevorzugt kleiner 5 Kelvin betrieben.

Das erfindungsgemäße Verfahren wird, bei der Hydrierung von aromatischen Carbonsäuren oder deren Derivaten, bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und/oder einer hohen Raum-Zeit-Ausbeute werden die in Schlaufenfahrweise betriebenen Reaktoren vorzugsweise mit hohen Flüssigkeitsbelastungen von 10 bis 400, bevorzugt von 20 bis 200 und besonders bevorzugt von 40 bis 150 m³ pro m² Querschnitt des leeren Reaktors und Stunde gefahren.
Die Flüssigkeitsbelastungen können in den in Schlaufenfahrweise betriebenen Reaktoren gleich oder verschieden sein. Vorzugsweise ist die Flüssigkeitsbelastung im ersten Reaktor am größten und nimmt in den nachfolgenden in Schlaufenfahrweise betriebenen Reaktoren ab. In einer Anlage gemäß der Erfindung mit zwei hintereinandergeschalteten Schlaufenreaktoren liegt die Flüssigbelastung, bei der Hydrierung von aromatischen Carbonsäuren oder deren Derivaten, im ersten Reaktor bevorzugt im Bereich von 20 bis 200, insbesondere im Bereich von 40 bis 150 m³/(m²*h) und im zweiten Reaktor bevorzugt im Bereich von 20 bis 180, insbesondere im Bereich von 40 bis 140 m³/(m²*h). Die Belastung des im geraden Durchgang betriebenen Reaktors beträgt vorzugsweise von 2 bis 100 m³/(m²*h), insbesondere von 10 bis 80 m³/(m²*h).

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Für die Hydrierung von aromatischen Carbonsäuren oder deren Derivaten können beispielsweise folgende Stoffe als Lösemittel eingesetzt werden: Geradkettige oder cyclische Ether, wie beispielsweise Tetrahydrofuran oder Dioxan sowie aliphatische Alkohole, in denen der Alkylrest 1 bis 13 Kohlenstoffatome aufweist. Bevorzugt als Lösemittel verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole. Die Verwendung von Alkoholen ist nur dann bevorzugt, wenn die zur Hydrierung vorgesehenen Verbindungen Carbonsäureester sind. Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Aromatengehalt im Reaktorzulauf zwischen 1 und 70 %, Der gewünschte Konzentrationsbereich kann bei denjenigen Reaktoren, die in Schlaufenfahrweise betrieben werden, durch das Kreislaufverhältnis (Mengenverhältnis von rückgeführten Hydrieraustrag zu Edukt) eingestellt werden. Die Aromatenkonzentrationen im Reaktorzulauf (Gemisch aus Frisch-Edukt oder Hydrieraustrag des vorigen Reaktors und aus Kreislaufstrom) nehmen vom ersten bis zum letzten Reaktor bevorzugt ab. Beispielweise liegen in einer Anlage nach Fig. 1 die Aromatenkonzentration im Zulauf zum ersten Reaktor (3) im Bereich von 70 bis 5 Massen-%, im Zulauf zum zweiten Reaktor (11) im Bereich von 40 bis 2 Massen-% und im Zulauf zum dritten Reaktor (18) im Bereich von 20 bis 1 Massen-%.

Das erfindungsgemäße Verfahren wird, bei der Hydrierung von aromatischen Carbonsäuren oder deren Derivaten, bevorzugt in einem Druckbereich von 0,3 bis 30 MPa, insbesondere von 1,5 bis 20 MPa, ganz besonders bevorzugt von 5 bis 20 MPa durchgeführt. Der Druck kann in den einzelnen Reaktoren gleich oder verschieden sein. Vorzugsweise sind die Drücke gleich oder annähernd gleich.

Die Hydriertemperaturen betragen, bei der Hydrierung von aromatischen Carbonsäuren oder deren Derivaten, bevorzugt von 50 bis 250 °C, vorzugsweise von 80 bis 200 °C. Die Hydriertemperaturen können in einzelnen Reaktoren gleich oder verschieden sein.

Als Produkte des erfindungsgemäßen Verfahrens werden entsprechende Zusammensetzungen erhalten, die von den Einsatzstoffen und dem Umsatz bei der Hydrierung abhängig sind. Die Zusammensetzung, die bei der erfindungsgemäßen Hydrierung einer aromatischen Carbonsäure oder deren Derivate, insbesondere eines aromatischen Di- oder Polycarbonsäureesters oder eines Gemisches aromatischer Di- oder Polycarbonsäureester nach dem erfindungsgemäßen Verfahren entsteht, hat bei Einsatz einer reinen aromatischen Carbonsäure oder deren Derivat, vorzugsweise einen Gehalt an alicyclischen Carbonsäuren oder deren Derivate, insbesondere Ester von über 96 Massen-%, insbesondere von über 98 Massen-%, ganz besonders bevorzugt von über 99 Massen-%. Dieses Gemisch kann direkt oder nach Reinigung eingesetzt werden. Die Abtrennung von Nebenprodukten kann beispielsweise durch Destillation oder durch Strippen mit einem Inertgas wie Stickstoff oder Wasserdampf erfolgen. Bevorzugt werden geringe Mengen an Leichtsieder durch Strippen mit Wasserdampf im Temperaturbereich von 120 °C bis 240 °C, insbesondere im Bereich von 150 bis 200 °C und bei einem Druck von 5 kPa bis 10 kPa abgetrennt. Anschließend kann durch Verringern des Druckes auf unter 5 kPa das Produkt getrocknet werden.

Als Produkte können mit dem erfindungsgemäßen Verfahren insbesondere Gemische bzw. Zusammensetzungen erhalten werden, die alicyclischen Carbonsäuren und/oder deren Derivate, insbesondere alicyclische Carbonsäureester und besonders bevorzugt alicyclische Di- oder Polycarbonsäuren aufweisen.

Die Verwendung der erfindungsgemäß hergestellten alicyclischen Carbonsäuren und/oder deren Derivate, insbesondere Ester aufweisende Zusammensetzungen können als Weichmacher in Kunststoffen Verwendungfinden. Bevorzugte Kunststoffe sind PVC, Homo- und Copolymere auf Basis von Ethylen, Propylen, Butadien, Vinylacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest und Isononylrest, Polymethacrylat, Polymethylmethacrylat, Methylacrylat-Butylacrylat-Copolymere, Methylmethacrylat-Butylmethacrylat-Copolymere, EthylenVinylacetat-Copolymere, chloriertes Polyethylen, Nitrilkautschuk, Acrylnitril-Butadien-StyrolCopolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.
Darüber hinaus können die erfindungsgemäß hergestellten alicyclischen Carbonsäureester zur Modifizierung von Kunststoffmischungen, beispielsweise der Mischung eines Polyolefins mit einem Polyamid, eingesetzt werden. Geeignete Kunststoffe sind die bereits genannten Verbindungen. Solche Gemische enthalten bevorzugt mindestens 5 Massen-%, besonders bevorzugt 10-80 Massen-%, ganz besonders bevorzugt 20-70 Massen-% der alicyclischen Polycarbonsäureester.

Gemische aus Kunststoffen, insbesondere PVC, die einen oder mehrere der erfindungsgemäß hergestellten alicyclischen Polycarbonsäureester enthalten, können beispielsweise in folgenden Produkten enthalten sein, bzw. zu deren Herstellung verwendet werden:
Schläuchen, Kabeln, Drahtummantelungen, Isolierbändern, im Fahrzeug- und Möbelbau, Plastisole, in Bodenbelägen, medizinische Artikel, Lebensmittelverpackungen, Dichtungen, Folien, Verbundfolien, Platten, Kunstleder, Spielzeug, Tapeten, Verpackungsbehälter, Klebebandfolien, Bekleidung, Beschichtungen, Beschichtungen von Geweben, Schuhen, Unterbodenschutz, Nahtabdichtungen, Modelliermassen oder Bällen.

Neben den obengenannten Anwendungen können die erfindungsgemäß hergestellten alicyclischen Carbonsäureester als Schmierölkomponente, als Bestandteil von Kühlflüssigkeiten und Metallbearbeitungsflüssigkeiten verwendet werden. Ebenso können sie als Komponente in Farben, Lacken, Tinten und Klebstoffen eingesetzt werden.

Das erfindungsgemäße Verfahren kann in verschiedenen Ausführungsformen durchgeführt werden. Eine bevorzugte Ausführungsformen der vorliegenden Erfindung ist als Blockschema in der Figur Fig. 1 beispielhaft dargestellt. Dieses Schema weist drei Reaktoren bzw. Reaktoreinheiten auf, von denen zwei in Schlaufenfahrweise betrieben werden. Es versteht sich von selbst, dass das erfindungsgemäße Verfahren auch mit mehr als drei Reaktoren (bzw. Reaktoreinheiten) durchgeführt werden kann oder dass alle drei Reaktoren in Schlaufenfahrweise betrieben werden können.

In der Variante des erfindungsgemäßen Verfahrens nach Fig. 1 werden Wasserstoff (1a), Edukt (2) und ein Teil (8) des flüssigen Hydrieraustrags (7) aus Reaktor (3) in die Hydriereinheit (3) eingespeist. Der Hydrieraustrag (4) aus der Hydriereinheit (3) wird in der Blase (5) in Abgas (6) und Flüssigphase (7) getrennt. Ein Teil (9) des Stroms (7) wird zusammen mit dem Teil (16) der Flüssigphase (15) aus der zweiten Hydriereinheit (11) und Wasserstoff (1b) in die Hydriereinheit (11)geleitet. Der Hydrieraustrag (12) aus der Hydriereinheit (11) wird in der Blase (13) in Abgas (14) und Flüssigphase (15) getrennt. Ein Teil (17) des Stroms (15) wird zusammen mit Wasserstoff (1c) in die Hydriereinheit (18) eingespeist. Der Hydrieraustrag (19) aus der Hydriereinheit (18) wird in der Blase (20) in Abgas (21) und Rohprodukt (22) getrennt. Rohprodukt (22) wird entweder als solches oder nach Aufreinigung in einer nicht dargestellten Anlage verwendet.

Die vorliegende Erfindung wird in den nachfolgenden Beispielen beispielhaft beschrieben, ohne dass die Erfindung auf die in den Beispielen angegebenen Ausführungsformen beschränkt sein soll.

### Beispiele

In den nachfolgenden Beispielen wird die Durchführung der Hydrierung beschrieben, wobei eine Endkonzentration von DINP im Ausgang der letzten Stufe von kleiner 0,05 % der Eingangskonzentration in der 1. Stufe erreicht werden sollte. In Beispiel 1 wurde die Hydrierung unter Verwendung von Parametern durchgeführt, die unter Einsatz von Katalysatorvolumina in den drei Stufen erhalten wurden, die erfindungsgemäß durch Ermittlung des Minimums des notwendigen Gesamtkatalysatorvolumens erhalten wurde. Im Vergleichsbeispiel (Beispiel 2) wurden Katalysatorvolumina eingesetzt, wie sie bei einer Abweichung von mehr als 20 % vom Minimum des ermittelten Gesamtkatalysatorvolumens erhalten werden.

Der Hydrierreaktor ist ein Rohrreaktor und wird wahlweise im geraden Durchgang oder in Schlaufenfahrweise kontinuierlich betrieben. Bei allen Versuchen strömen die flüssige Phase und das Hydriergas im Gleichstrom von oben nach unten.

Der Rohrreaktor ist mit 1350 ml Ruthenium-Katalysator (1 % Ru/TiO₂) gefüllt. Dieser Katalysator wird aus dem TiO₂-Träger Aerolyst 7711 und einer wässrigen Rutheniumnitrat-Lösung, wie in DE 102 32 868.4 beschrieben, hergestellt. Der Katalysator besteht aus zylindrischen Strangextrudaten mit dem Kreisdurchmesser von 1,5 mm und einer Länge von 4 bis 6 mm.

In beiden Versuchen wird Diisononylphthalat, abgekürzte Schreibweise DINP, der Oxeno Olefinchemie GmbH mit der Markenbezeichnung Vestinol 9 eingesetzt. Als Hydriergas wird Wasserstoff in einer Reinheit von über 99,9 % eingesetzt.

Bei beiden Beispielen ist der flüssige Hydrieraustrag einer Hydrierstufe das Einsatzprodukt der nächsten Hydrierstufe. Die einzelnen Hydrierstufen werden nacheinander im gleichen Reaktor mit dem gleichen Katalysator und gleicher Katalysatormenge durchgeführt. Der Hydrieraustrag der ersten Stufe wird nach Erreichen des quasistationären Gleichgewichts gesammelt und für die zweite Stufe verwendet. Zusätzlich wird der Hydrieraustrag der zweiten Stufe als Einsatzmaterial für die dritte Stufe gesammelt. Zur besseren Vergleichbarkeit war der Druck, die Reaktionstemperatur und die Abgasmenge in allen Hydrierschritten gleich.

### Beispiel 1 (erfindungsgemäß)

Die Hydrierung wird in drei Stufen durchgeführt. In den ersten beiden Stufen wird der Reaktor in Schlaufenfahrweise und in der dritten Stufe im geraden Durchgang betrieben.

Die Betriebsparameter und die Massenströme von Beispiel 1 werden in Tabelle 1 zusammengestellt.

**Tabelle 1:**

| | 1. Stufe | 2. Stufe | 3. Stufe |
|---|---|---|---|
| Temperatur (°C) | 100 | 100 | 100 |
| Druck (MPa) | 10 | 10 | 10 |
| Abgas (Nl/h) | 50 | 50 | 50 |
| Kreislaufmenge (l/h) | 30 | 30 | 0 |
| Zulauf (l/h) | 9,54 | 3,18 | 1,05 |
| DINP-Konzentration im Zulauf (%) (bezogen auf Frisch-DINP oder Hydrieraustrag des vorherigen Reaktors) | 100 | 59,7 | 19,9 |
| Hydrieraustrag (l/h)* | 9,592 | 3,197 | 1,053 |
| DINP-Konzentration im Hydrieraustrag (%) | 59,7 | 19,9 | < 0,05 |
| LHSV (h⁻¹) ** | 7,06 | 2,35 | 0,78 |

| | | | |
|---|---|---|---|
| * Volumen des Hydrieraustrags, berechnet unter Zugrundelegung einer Dichte von 0,975 g/l und unter Vernachlässigung der Abgasverluste ** LHSV : Liter Frisch-DINP oder Liter Hydrieraustrag aus dem vorherigen Reaktor je Liter Katalysator je Stunde | | | |

Das Zielprodukt (Hydrieraustrag der dritten Stufe) weist eine Reinheit von über 99,5 Massen-% auf. Der DINP-Umsatz ist praktisch quantitativ.

Unter Berücksichtigung, dass der Hydrieraustrag einer Stufe das Einsatzmaterial der nächsten Stufe ist, ergäben sich, ausgehend von 9,54 1/h Frisch-DINP, für eine kontinuierliche Hydrierung unter Berücksichtigung der unterschiedlichen Dichten von DINP bzw. DINCH folgende Zulaufströme (ohne Rückführströme) zu den Reaktoren:
Erster Reaktor: 9,54 l/h
Zweiter Reaktor: 9,5921/h
Dritter Reaktor: 9,643 l/h

Unter Beibehaltung der in Tabelle 1 ausgewiesenen LHSV ergäben sich für die gerade genannten Volumenströmen folgende Katalysatorvolumina:
Erster Reaktor: 1,351
Zweiter Reaktor : 4,07 1
Dritter Reaktor: 12,361

Das (Gesamt-)Katalysatorvolumen in den beiden Schlaufenreaktoren entspricht demnach 5,42 1. Für die gesamte Hydrierung sind 17,78 1 Katalysator erforderlich. Bei der Zuführung von 9,54 1/h Frisch-DINP zum ersten Reaktor ergab sich über die beiden Schlaufenreaktoren eine Gesamt-LHSV von 1,75 h⁻¹.

### Beispiel 2 (Vergleich)

Die Hydrierung wird in drei Stufen durchgeführt. In den ersten beiden Stufen wird der Reaktor in Schlaufenfahrweise und in der dritten Stufe im geraden Durchgang betrieben.

Die Betriebsparameter und die Massenströme von Beispiel 2 werden in Tabelle 2 zusammengestellt.

**Tabelle 2:**

| | 1. Stufe | 2. Stufe | 3. Stufe |
|---|---|---|---|
| Temperatur (°C) | 100 | 100 | 100 |
| Druck (MPa) | 10 | 10 | 10 |
| Abgas (Nl/h) | 50 | 50 | 50 |
| Kreislaufinenge (l/h) | 30 | 30 | 0 |
| Zulauf (l/h) | 9,54 | 1,28 | 1,20 |
| DINP-Konzentration im Zulauf (%) (bezogen auf Frisch-DIMP oder Hydrieraustrag des vorherigen Reaktors) | 100 | 59,7 | 10,1 |
| Hydrieraustrag (l/h)* | 9,592 | 1,29 | 1,20 |
| DINP-Konzentration im Hydrieraustrag (%) | 59,7 | 10,1 | <0,05 |
| LHSV (h⁻¹) ** | 7,06 | 0,95 | 0,88 |

| | | | |
|---|---|---|---|
| * Volumen des Hydrieraustrags, berechnet unter Zugrundelegung einer Dichte von 0,975 g/l und unter Vernachlässigung der Abgasverluste ** LHSV: Liter Frisch-DINP oder Liter Hydrieraustrag aus dem vorherigen Reaktor je Liter Katalysator je Stunde | | | |

Das Zielprodukt (Hydrieraustrag der dritten Stufe) hat eine Reinheit von über 99,5 Massen-%. Der DINP-Umsatz ist praktisch quantitativ.

Unter Berücksichtigung, dass der Hydrieraustrag einer Stufe das Einsatzmaterial der nächsten Stufe ist, ergäben sich, ausgehend von 9,54 1/h Frisch-DINP, für eine kontinuierliche Hydrierung unter Berücksichtigung der unterschiedlichen Dichten von DINP bzw. DINCH folgende Zulaufströme (ohne Rückführströme) zu den Reaktoren:
Erster Reaktor: 9,54 l/h
Zweiter Reaktor: 9,592 l/h
Dritter Reaktor: 9,643 l/h

Unter Beibehaltung der in Tabelle 2 ausgewiesenen LHSV ergäben sich für die gerade genannten Volumenströmen folgende Katalysatorvolumina:
Erster Reaktor: 1,35 l
Zweiter Reaktor: 10,09 1
Dritter Reaktor: 10,96 1

Das (Gesamt-)Katalysatorvolumen in den beiden Schlaufenreaktoren entsprach demnach 11,44 l. Für die gesamte Hydrierung sind 22,4 l Katalysator erforderlich. Bei der Zuführung von 9,54 1/h Frisch-DINP zum ersten Reaktor ergibt sich über die beiden Schlaufenreaktoren eine Gesamt-LHSV von 0,43 h⁻¹.

Wie den Ergebnissen von Beispiel und Vergleichsbeispiel entnommen werden kann, ist es mit dem erfindungsgemäßen Verfahren möglich, das minimal notwendige Katalysatorvolumen für Hydrierreaktionen in zwei oder mehr hintereinandergeschalteten Reaktoren zu ermitteln, ohne dass der Gesamtumsatz beeinträchtigt wird.

## Patentansprüche

1. Verfahren zur kontinuierlichen katalytischen Hydrierung aromatischer Carbonsäuren oder deren Derivate oder von Gemischen aus zwei oder mehreren Carbonsäuren oder Carbonsäurederivaten an festen im Festbett angeordneten Katalysatoren mit einem wasserstoffhaltigen Gas,
**dadurch gekennzeichnet,**
**dass** die Hydrierung in mindestens drei hintereinander geschalteten Hydriereinheiten durchgeführt wird, wobei zumindest die beiden ersten Hydriereinheiten in Schlaufenfahrweise betrieben werden, wobei für die Hydriereinheiten Katalysatorvolumina verwendet werden, die um maximal 20 % von den Katalysatorvolumina abweichen, die erhalten sind durch
a) Bestimmung der Kinetik der durchzuführenden Hydrierung,
b) Berechnen des erforderlichen Katalysatorvolumens für die eingesetzten Reaktortypen bei vorgegebenen Reaktorein- und -ausgangskonzentrationen,
c) Ermitteln des erforderlichen Gesamtkatalysatorvolumens durch Kombination der berechneten Katalysatorvolumina, wobei jeweils nur solche Kombinationen vorgenommen werden, die zur gewünschten Endkonzentration des eingesetzten zu hydrierenden Eduktes führen,
d) Erstellen einer Kurve aus den in c) ermittelten Gesamtkatalysatorvolumina aufgetragen gegen den Umsatz,
e) Bestimmen des Minimums der in d) erstellten Kurve und
f) Ermitteln der dem Minimum zuzuordnenden Katalysatorvolumina der einzelnen Hydriereinheiten.

2. Verfahren nach Anspruch 1,
**dadurch gekennzeichnet,**
**dass** alle Hydriereinheiten in Schlaufenfahrweise betrieben werden.

3. Verfahren nach Anspruch 1,
**dadurch gekennzeichnet,**
**dass** die letzte Hydriereinheit im geraden Durchgang betrieben wird.

4. Verfahren nach einem der Ansprüche 1 bis 3,
**dadurch gekennzeichnet,**
**dass** die Hydrierung in einer Vorrichtung durchgeführt wird, die drei Hydriereinheiten aufweist.

5. Verfahren nach einem der Ansprüche 1 bis 4,
**dadurch gekennzeichnet,**
**dass** ein Katalysator, der mindestens ein Metall der achten Nebengruppe des Periodensystems der Elemente enthält, verwendet wird.

6. Verfahren nach Anspruch 5,
**dadurch gekennzeichnet,**
**dass** ein Katalysator eingesetzt wird, der Ruthenium enthält.

7. Verfahren nach einem der Ansprüche 1 bis 6,
**dadurch gekennzeichnet,**
**dass** ein Katalysator eingesetzt wird, der einen Titandioxid-Träger aufweist.

8. Verfahren nach Anspruch 1,
**dadurch gekennzeichnet,**
**dass** als aromatische Carbonsäuren oder deren Derivate Mono-, Di- oder Polycarbonsäuren oder deren Derivate eingesetzt werden.

9. Verfahren nach Anspruch 8,
**dadurch gekennzeichnet,**
**dass** als aromatische Di- oder Polycarbonsäuren oder deren Derivate Benzol-, Diphenyl-, Naphthalin-, Diphenyloxid- oder Anthracen-Di- oder Polycarbonsäuren, deren Anhydride und/oder deren entsprechende Ester eingesetzt werden.

10. Verfahren nach Anspruch 8 oder 9,
**dadurch gekennzeichnet,**
**dass** als aromatische Monocarbonsäure Benzoesäure, 1-Naphthoesäure oder 2-Naphthoesäure, deren Anhydride oder deren Ester eingesetzt werden.

11. Verfahren nach zumindest einem der Ansprüche 8 bis 10,
**dadurch gekennzeichnet,**
**dass** als aromatische Carbonsäurederivate die Carbonsäureester eingesetzt werden.

12. Verfahren nach Anspruch 11,
**dadurch gekennzeichnet,**
**dass** bei Einsatz von Di- oder Polycarbonsäurederivaten die Partial- und/oder Vollester eingesetzt werden.

13. Verfahren nach Anspruch 11 oder 12
**dadurch gekennzeichnet,**
**dass** die Alkoholkomponenten der eingesetzten aromatischen Carbonsäureester verzweigte oder unverzweigte Alkoxyalkyl-, Cycloalkyl- und/oder Alkylgruppen mit 1 bis 25 Kohlenstoffatomen aufweisen.

14. Verfahren nach zumindest einem der Ansprüche 11 bis 13,
**dadurch gekennzeichnet,**
**dass** die Alkoholkomponenten der eingesetzten aromatischen Di- und/oder Polycarbonsäureester jeweils gleich oder unterschiedlich sind.

15. Verfahren nach zumindest einem der Ansprüche 11 bis 13,
**dadurch gekennzeichnet,**
**dass** als aromatisches Dicarbonsäurederivat Diisononylphthalat oder Didecylphthalat eingesetzt wird.

16. Verfahren nach Anspruch 10,
**dadurch gekennzeichnet,**
**dass** als aromatisches Monocarbonsäurederivat Isononylbenzoat oder Decylbenzoat eingesetzt wird.

17. Verfahren nach zumindest einem der Ansprüche 1 bis 16,
**dadurch gekennzeichnet,**
**dass** für die Schlaufenreaktoren Katalysatorvolumina verwendet werden, die um maximal 5 % von den ermittelten Katalysatorvolumina abweichen.

## Claims

1. Method for the continuous catalytic hydrogenation of aromatic carboxylic acids or their derivatives or of mixtures of two or more carboxylic acids or carboxylic acid derivatives using a hydrogen-containing gas in the presence of solid catalysts disposed in a fixed bed, **characterized in that** the hydrogenation is carried out in at least three series-connected hydrogenation units, wherein at least the first two hydrogenation units are operated in loop operating mode, with, in the hydrogenation units, catalyst volumes being used which deviate by a maximum of 20% from the catalyst volumes which are obtained by
a) determining the kinetics of the hydrogenation to be carried out,
b) calculating the required catalyst volume for the reactor types used for preset reactor input and output concentrations,
c) determining the required total catalyst volume by combining the calculated catalyst volumes, in each case only those combinations being performed which lead to the desired end concentration of the starting material to be hydrogenated used,
d) preparing a curve from the total catalyst volumes determined in c) plotted against the conversion rate,
e) determining the minimum of the curve prepared in d) and
f) determining the catalyst volumes, of the individual hydrogenation units, to be assigned to the minimum.

2. Method according to Claim 1,
**characterized in that**
all hydrogenation units are operated in loop operating mode.

3. Method according to Claim 1,
**characterized in that**
the last hydrogenation unit is operated in straight through-flow passage.

4. Method according to one of Claims 1 to 3,
**characterized in that**
the hydrogenation is carried out in an apparatus which has three hydrogenation units.

5. Method according to one of Claims 1 to 4,
**characterized in that**
use is made of a catalyst which comprises at least one metal of the eighth subgroup of the Periodic Table of the Elements.

6. Method according to Claim 5,
**characterized in that**
use is made of a catalyst which comprises ruthenium.

7. Method according to one of Claims 1 to 6,
**characterized in that**
use is made of a catalyst which has a titanium dioxide support.

8. Method according to Claim 1,
**characterized in that**
as aromatic carboxylic acids or their derivatives, use is made of mono-, di- or polycarboxylic acids or their derivatives.

9. Method according to Claim 8,
**characterized in that**
as aromatic di- or polycarboxylic acids or their derivatives, use is made of benzene-, diphenyl-, naphthalene-, diphenyl oxide- or anthracene di- or polycarboxylic acids, their anhydrides, and/or their corresponding esters.

10. Method according to Claim 8 or 9,
**characterized in that**
as aromatic monocarboxylic acid, use is made of benzoic acid, 1-naphthoic acid, or 2-naphthoic acid , their anhydrides or their esters.

11. Method according to at least one of Claims 8 to 10,
**characterized in that**
as aromatic carboxylic acid derivatives, use is made of the carboxylic esters.

12. Method according to Claim 11,
**characterized in that**
when di- or polycarboxylic acid derivatives are used, the partial and/or full esters are used.

13. Method according to Claim 11 or 12,
**characterized in that**
the alcohol components of the aromatic carboxylic esters used have branched or unbranched alkoxyalkyl, cycloalkyl and/or alkyl groups having 1 to 25 carbon atoms.

14. Method according to at least one of Claims 11 to 13,
**characterized in that**
the alcohol components of the aromatic di- and/or polycarboxylic esters used are in each case identical or different.

15. Method according to at least one of Claims 11 to 13,
**characterized in that**
as aromatic dicarboxylic acid derivative, use is made of diisononyl phthalate or didecyl phthalate.

16. Method according to Claim 10,
**characterized in that**
as aromatic monocarboxylic acid derivative, use is made of isononyl benzoate or decyl benzoate.

17. Method according to at least one of Claims 1 to 16,
**characterized in that**
for the loop reactors, catalyst volumes are used which deviate by a maximum of 5% from the catalyst volumes determined.

## Revendications

1. Procédé pour l'hydrogénation catalytique continue d'acides carboxyliques aromatiques ou de leurs dérivés ou de mélanges de deux ou plus de deux acides carboxyliques ou dérivés d'acides carboxyliques sur des catalyseurs solides disposés en lit fixe, avec un gaz contenant de l'hydrogène,
**caractérisé en ce qu'**on effectue l'hydrogénation dans au moins trois unités d'hydrogénation successives, en faisant fonctionner au moins les deux premières unités d'hydrogénation en mode opératoire à circulation, en utilisant pour les unités d'hydrogénation des volumes de catalyseur qui diffèrent d'au maximum 20 % des volumes de catalyseur qui sont obtenus par
a) détermination de la cinétique de l'hydrogénation à effectuer,
b) calcul du volume nécessaire de catalyseur pour les types de réacteurs utilisés aux concentrations préétablies à l'entrée et à la sortie des réacteurs,
c) détermination des volumes totaux de catalyseur requis par combinaison des volumes de catalyseur calculés, en ne faisant chaque fois que les combinaisons qui conduisent à la concentration finale désirée du produit de départ à hydrogéner qui est utilisé,
d) construction d'une courbe à partir des volumes totaux de catalyseur déterminés en c), portés en fonction de la conversion,
e) détermination du minimum de la courbe construite en d) et
f) détermination des volumes de catalyseur, à affecter au minimum, des unités d'hydrogénation individuelles.

2. Procédé selon la revendication 1, **caractérisé en ce qu'**on fait fonctionner toutes les unités d'hydrogénation en mode opératoire à circulation.

3. Procédé selon la revendication 1, **caractérisé en ce qu'**on fait fonctionner la dernière unité d'hydrogénation en passage direct.

4. Procédé selon l'une quelconque des revendications 1 à 3, **caractérisé en ce qu'**on effectue l'hydrogénation dans un dispositif qui comporte trois unités d'hydrogénation.

5. Procédé selon l'une quelconque des revendications 1 à 4, **caractérisé en ce qu'**on utilise un catalyseur qui contient au moins un métal du groupe VIIIB du système périodique des éléments.

6. Procédé selon la revendication 5, **caractérisé en ce qu'**on utilise un catalyseur qui contient du ruthénium.

7. Procédé selon l'une quelconque des revendications 1 à 6, **caractérisé en ce qu'**on utilise un catalyseur qui comporte un support en dioxyde de titane.

8. Procédé selon la revendication 1, **caractérisé en ce qu'**on utilise comme acides carboxyliques aromatiques ou leurs dérivés des acides mono-, di- ou polycarboxyliques ou leurs dérivés.

9. Procédé selon la revendication 8, **caractérisé en ce qu'**on utilise comme acides di- ou polycarboxyliques aromatiques ou leurs dérivés les acides benzène-, diphényl-, naphtalène-, diphényloxy- ou anthracène-di- ou polycarboxyliques, leurs anhydrides et/ou leurs esters correspondants.

10. Procédé selon la revendication 8 ou 9, **caractérisé en ce qu'**on utilise comme acide monocarboxylique aromatique l'acide benzoïque, l'acide 1-naphtoïque ou l'acide 2-naphtoïque, leurs anhydrides ou leurs esters.

11. Procédé selon au moins l'une des revendications 8 à 10, **caractérisé en ce qu'**on utilise comme dérivés d'acides carboxyliques aromatiques les esters d'acides carboxyliques.

12. Procédé selon la revendication 11, **caractérisé en ce que** dans le cas d'utilisation de dérivés d'acides di- ou polycarboxyliques, on utilise les esters partiels et/ou les esters totaux.

13. Procédé selon la revendication 11 ou 12, **caractérisé en ce que** les composants alcool des esters d'acides carboxyliques aromatiques utilisés comportent des groupes alcoxyalkyle, cycloalkyle et/ou alkyle ramifiés ou non ramifiés, ayant de 1 à 25 atomes de carbone.

14. Procédé selon au moins l'une des revendications 11 à 13, **caractérisé en ce que** les composants alcool des esters d'acides di- et/ou polycarboxyliques aromatiques utilisés sont chaque fois identiques ou différents.

15. Procédé selon au moins l'une des revendications 11 à 13, **caractérisé en ce qu'**on utilise comme dérivé d'acide dicarboxylique aromatique le phtalate de diisononyle ou le phtalate de didécyle.

16. Procédé selon la revendication 10, **caractérisé en ce qu'**on utilise comme dérivé d'acide monocarboxylique aromatique le benzoate d'isononyle ou le benzoate de décyle.

17. Procédé selon au moins l'une des revendications 1 à 16, **caractérisé en ce que** pour les réacteurs à circulation on utilise des volumes de catalyseur qui diffèrent d'au maximum 5 % des volumes de catalyseur déterminés.
